(19) Europäisches Patentamt
European Patent Office
Office européen des brevets

(11) Veröffentlichungsnummer: **0 377 909 A1**

(12) **EUROPÄISCHE PATENTANMELDUNG**

(21) Anmeldenummer: **89124179.6**

(51) Int. Cl.$^5$: **C07D 303/30, C08G 59/04**

(22) Anmeldetag: **30.12.89**

(30) Priorität: **12.01.89 DE 3900682**

(43) Veröffentlichungstag der Anmeldung:
**18.07.90 Patentblatt 90/29**

(84) Benannte Vertragsstaaten:
**BE CH DE ES FR GB IT LI NL**

(71) Anmelder: **BAYER AG**

**D-5090 Leverkusen 1 Bayerwerk(DE)**

(72) Erfinder: **Müller, Hanns-Peter, Dr.**
**Weizenfeld 36**
**D-5060 Bergisch Gladbach 2(DE)**
Erfinder: **Franke, Joachim, Dr.**
**An der Ruthen 2**
**D-5000 Köln 80(DE)**

(54) **Diglycidylverbindungen, ihre Herstellung und ihre Verwendung in härtbaren Epoxid-Gemischen.**

(57) Diglycidylverbindungen von gegebenenfalls ringsubstituierten Dihydroxydiphenyl-cycloalkanen sowie höhermolekularer Bisepoxide dieser Struktur, ferner Epoxidharzgemische, enthaltend die neuen Diglycidylverbindungen sowie die Verwendung der neuen Diglycidylverbindungen und deren Epoxidharzgemische in härtbaren Mischungen.

EP 0 377 909 A1

## Diglycidylverbindungen, ihre Herstellung und ihre Verwendung in härtbaren Epoxid-Gemischen

Gegenstand der Erfindung sind Diglycidylverbindungen der Formel (I)

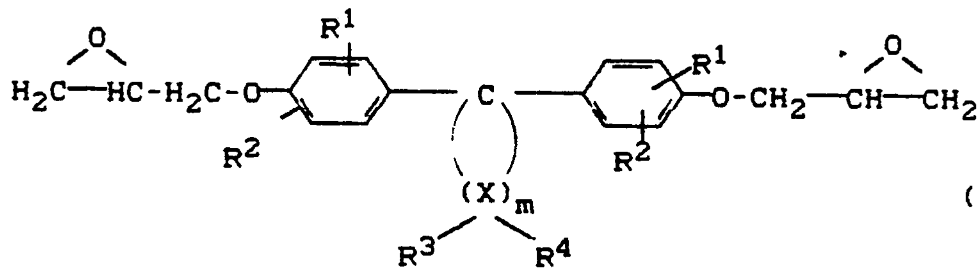

(I)

worin
$R^1$ und $R^2$ unabhängig voneinander Wasserstoff, Halogen, $C_1$-$C_8$-Alkyl, $C_5$-$C_6$-Cycloalkyl, $C_6$-$C_{10}$-Aryl und $C_7$-$C_{12}$-Aralkyl,
m eine ganze Zahl von 4 bis 7,
$R^3$ und $R^4$ für jedes X individuell wählbar, unabhängig voneinander Wasserstoff, $C_1$-$C_6$-Alkyl,
X Kohlenstoff bedeuten,
mit der Maßgabe, daß an mindestens einem Atom X, $R^3$ und $R^4$ gleichzeitig Alkyl ist sowie höhermolekulare Bisepoxide dieser Struktur.

Ein weiterer Gegenstand der Erfindung ist ein Verfahren zur Herstellung der Diglycidylverbindungen der Formel (I), das dadurch gekennzeichnet ist, daß man gegebenenfalls ringsubstituierte Diphenole der Formel (II)

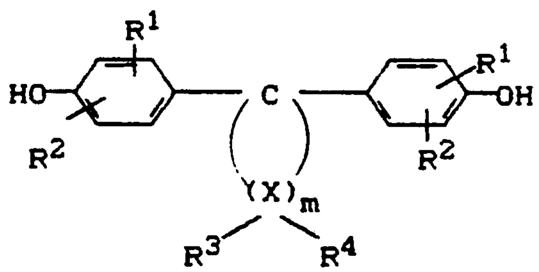

(II)

worin
$R^1$, $R^2$, $R^3$, $R^4$, X und m die gleiche Bedeutung wie in Formel (I) haben,
mit überschüssigem Epichlorhydrin in Gegenwart von Katalysatoren und Alkaliverbindungen in an sich bekannter Weise umsetzt.

Gegenstand der vorliegenden Erfindung sind schließlich Mischungen, enthaltend Diglycidylverbindungen der Formel (I), sowie höhermolekulare Bisepoxide dieser Struktur und gegebenenfalls bekannte Di-, Tri- und Tetra-glycidylverbindungen. Wenn diese Mischungen Härtungsmittel für Epoxidharze enthalten, sind sie - wie andere Epoxidmischungen - härtbar.

Die Mischungen enthalten Vorzugsweise mindestens 5 Gew.-%, vorzugsweise mindestens 20 Gew.-% der Diglycidylverbindungen der Formel (I). Die Mischungen ergeben Härtungsprodukte mit besseren physikalischen Eigenschaften.

Die Glycidylierung von Hydroxylgruppen aufweisenden organischen Verbindungen ist grundsätzlich bekannt. Bisphenole der Formel (II) können beispielsweise mit Epichlorhydrin zu einem Dichlorhydrin der Formel (III) umgesetzt werden, das dann dehydrohalogeniert wird.

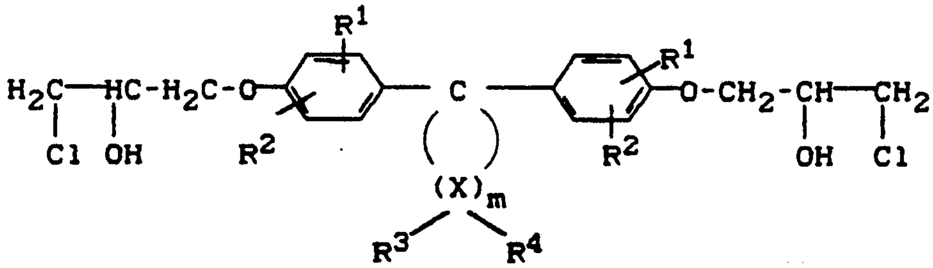

(III)

Man arbeitet vorzugsweise wie folgt:

In einer ersten Stufe wird 1 Mol Bisphenol mit wenigstens 10 Mol, vorzugsweise 20 bis 40 Mol Epichlorhydrin in Gegenwart eines Katalysators (z.B. Tetraalkylammoniumchlorid) umgesetzt, wobei ein Dichlorhydrin der Formel (III) entsteht. Im Gleichgewicht bilden sich schon während der Reaktion Glycidether und 1,3-Dichlorisopropanol durch Umsetzung von 3-Chlor-2-hydroxypropylethergruppen mit Epichlorhydrin. In einer zweiten Stufe wird der Bischlorhydrinether (III) mit Alkali behandelt, wobei die Expoxidgruppen gebildet werden und das entstandene 1,3-Dichlorisopropanol in Epichlorhydrin rücküberführt wird

Das Alkali ist normalerweise Natriumhydroxid, doch können auch andere alkalische Substanzen wie Bariumhydroxid oder Kaliumcarbonat für die Umwandlung der 1,2-Chlorhydringruppen in die 1,2-Epoxidgruppen verwendet werden.

Die Umsetzung kann auch in einem Lösungsmittel, beispielsweise Kohlenwasserstoff, Ether oder Keton durchgeführt werden, doch wird vorzugsweise ein Überschuß an Epichlorhydrin als Lösungsmittel verwendet. Die Umsetzung wird bei erhöhter Temperatur, etwa bei 40 bis 100 ° C, durchgeführt.

Vorzugsweise verwendet man als Verbindungen der Formel (II) solche, worin an 1-2 Atomen X, insbesondere nur an einem Atom X, $R^3$ und $R^4$ gleichzeitig Alkyl sind. Bevorzugter Alkylrest ist Methyl; die X-Atome in α-Stellung zu einem di-phenyl-substituierten C-Atom (C-1) sind bevorzugt nicht mit Alkyl substituiert, dagegen ist die Alkyl-di-substitution in ß-Stellung zu C-1 bevorzugt.

Besonders bevorzugt als Ausgangsmaterial sind Dihydroxydiphenylcycloalkane der Formeln

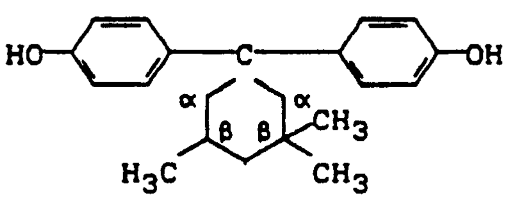

(IV)

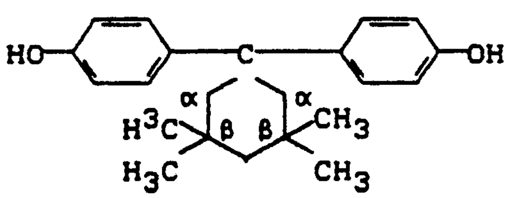

(V) und

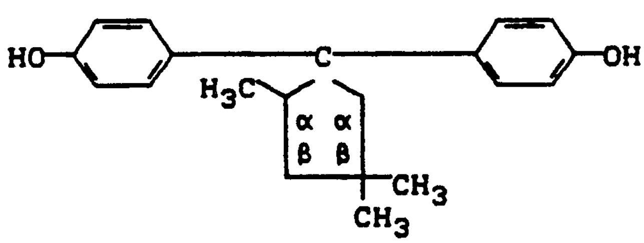

(VI),

wobei das 1,1-Bis-(4-hydroxyphenyl)-3,3,5-trimethylcyclohexan (Formel IV) besonders bevorzugt ist.

Bisphenole der Formel (II) und ihre Herstellung sind in der deutschen Patentanmeldung P 3 832 396.4 beschrieben.

Die erfindungsgemäßen Epoxidharzgemische mit an sich bekannten Di-, Tri- und Tetraglycidylverbindungen werden vozugsweise durch einfaches Mischen einer Diglycidylverbindung der Formel (I) mit den bekannten Epoxidharzen, wie sie z.B. in Polymere Werkstoffe, Band III, Technologie 2, herausgegeben von H. Batzer, Seite 171 bis 174, Georg Thieme Verlag, Stuttgart/New York, 1984 beschrieben sind, hergestellt.

Eine andere Möglichkeit zur Herstellung der erfindungsgemäßen Epoxidharzgemische besteht in der Glycidylierung eines Gemisches aus Bisphenolen der Formel (II) und z.B. Bisphenol A, aromatischen Diaminen, Aminophenolen oder Heterocyclen wie z.B. Cyanursäure.

Die erfindungsgemäßen Diglycidylverbindungen und deren Gemische mit bekannten Epoxidharzen können mit den üblichen Härtungsmitteln für Expoxidharze ausgehärtet werden. Besonders bevorzugt ist die Härtung der Diglycidylverbindungen (Formel 1) sowie höhermolekularer Bisepoxide dieser Struktur mit dem Bisphenol (Formel II) oder beliebigen Mischungen üblicher Härtungsmittel mit dem Bisphenol II.

Als Beispiele für Hartungsmittel seien die gebräuchlichen Härtungsmittel für Expoxidharze genannt, ein schließlich der aliphatischen, cycloaliphatischen, aromatischen und heterocyclischen Amine, wie Bis-(4-aminophenyl)-methan, Anilin-Formaldehyd-Harz, Bis-(4-aminophenyl)-sulfon, Propan-1,3-diamin, Hexamethylendiamin, Diethylentriamin, Triethylentetramin, 2,2,4-Trimethylhexan-1,6-diamin, m-Xylylendiamin, Bis-(4-aminocyclohexyl)-methan, 2,2-Bis-(4-aminocyclohexyl)propan und 3-Aminomethyl-3,5,5-trimethylcyclohexylamin (Isophorondiamin), Polyaminoamide, wie beispielsweise solche aus aliphatischen Polyaminen und dimerisierten oder trimerisierten Fettsäuren, Polyphenole, wie Resorcin, Hydrochinon, 2,2-Bis-(4-hydroxyphenyl)-propan und Phenol-Aldehyd-Harze, Polythiol, wie die im Handel unter der Bezeichnung "Thiokole" erhältlichen Polythiole, Polycarbonsäuren und ihre Anhydride, wie z.B. Phthalsäureanhydrid, Tetrahydrophthalsäureanhydrid, Hexahydrophthalsäureanhydrid, Hexachlorendomethylentetrahydrophthalsäureanhydrid, Pyromellitsäureanhydrid, Benzophenon-3,3′, 4,4′-tetracarbonsäuredianhydrid, die Säuren der zuvorgenannten Anhydride sowie auch Isophthalsäure und Terephthalsäure. Es können auch katalytisch wirkende Härtungsmittel verwendet werden, wie beispielsweise tertiäre Amine [z.B. 2,4,6-Tris-(dimethylaminoethyl)-phenol)], Imidazole und andere Mannichbasen; Alkalimetallalkoxide von Alkoholen (z.B. Na-Alkoholat von 2,4-Dihydroxy-3-hydroxymethyl-pentan), Zinnsalze von Alkansäuren (z.B. Zinnoctanoat), Friedel-Crafts-Katalysatoren, wie Bortrifluorid und Bortrichlorid und ihre Komplexe und Chelate, die durch Umsetzung von Bortrifluorid mit z.B. 1,3-Diketonen erhalten werden.

Mit den Härtungsmitteln können auch die geeigneten Härtungsbeschleuniger eingesetzt werden. Bei Verwendung von Poly-(aminoamiden), Polythiolen oder Polycarbonsäureanhydriden können tertiäre Amine oder deren Salze, quaternäre Ammoniumverbindungen oder Alkalimetallalkoxide als Beschleuniger dienen.

Die Menge des eingesetzten Härtungsmittels richtet sich nach der chemischen Natur des Härtungsmittels und nach den gewünschten Eigenschaften der härtbaren Mischung und des gehärteten Produktes. Die maximale Menge kann leicht ermittelt werden. Wenn das Hartungsmittel ein Amin ist, werden normalerweise 0,75 bis 1,25 äquivalente Aminwasserstoffe pro 1 Expoxidäquivalent eingesetzt. Wenn Polycarbonsäuren oder ihre Anhydride eingesetzt werden, verwendet man gewöhnlich 0,4 bis 1,1 äquivalente Carboxylgruppe bzw. Anhydridgruppe per 1 Äquivalent Epoxidgruppe. Bei der Verwendung von Polyphenolen als Härtungsmittel setzt man zweckmäßig 0,75 bis 1,25 phenolische Hydroxylgruppen per 1 Epoxidäquivalent ein.

Katalytisch wirkende Härtungsmittel werden allgemein in Mengen von 1 bis 40 Gew.-Teilen pro 100 Gew.-Teile Epoxidharz eingesetzt.

Je nach Natur des verwendeten Härtungsmittel kann die Härtung bei Raumtemperatur oder bei höheren Temperaturen vorgenommen werden. Die Härtung kann gewünschtenfalls auch in zwei Stufen vorgenommen werden, indem man z.B. den Härtungsvorgang unterbricht oder, falls man ein Härtungsmittel für höhere Temperaturen einsetzt, die härtbare Mischung bei tieferen Temperaturen teilweise härten läßt. Die dabei erhaltenen Produkte sind noch schmelzbare und lösliche Präkondensate (sogenannte "B-Stufenharze") und eignen sich z.B. für Pressmassen, Sinterpulver oder Prepregs.

Die erfindungsgemäßen härtbaren Mischungen können ferner noch Plastifizierungsmittel, wie Dibutylphthalat, Dioctylphthalat oder Trikresylphosphat, oder Additive erhalten, wie Streckmittel, Füllstoffe, Verstärkungsmittel, Färbemittel, Fließmittel und Formtrennmittel. Geeignete Streckmittel, Füllstoffe und Verstärkungsmittel sind beispielsweise Asbest, Asphalt, Bitumen, Glasfasern, Textilfasern, Kohlenstoff- oder Borfasern, Glimmer, Tonerde, Gips, Titandioxid, Kreide, Quarzmehl, Cellulose, Kaolin, gemahlener Dolomit, Wollastonit, Kieselerde mit großer spezifischer Oberfläche (erhältlich unter dem Handelsnamen "Aerosil®"), mit langkettigen Aminen modifizierte Tonerde (erhältlich unter dem Handelsnamen "Bentone®"), gepulvertes Polyvinylchlorid, Polyolefin oder Aminoplaste, Metallpulver, wie Aluminium- oder Eisenpulver. Flammschutzmittel, wie Antimontrioxid, können ebenfalls den hartbaren Mischungen zugegeben werden.

Die erfindungsgemäßen hartbaren Massen lassen sich z.B. als Laminier-, Tränk- und Gießharze, Pulverbeschichtungen, Formmassen, Kitte und Dichtungsmassen, Einbettungs- und Isoliermassen für die Elektrotechnik und insbesondere als Klebestoffe oder Matrixharze zur Herstellung von faserverstärkten Kunststoffen verwenden.

Beispiele

Beispiel 1

Herstellung von 1,1-Bis-(4-epoxypropoxyphenyl)-3.3.5-trimethylcyclohexan (VII).

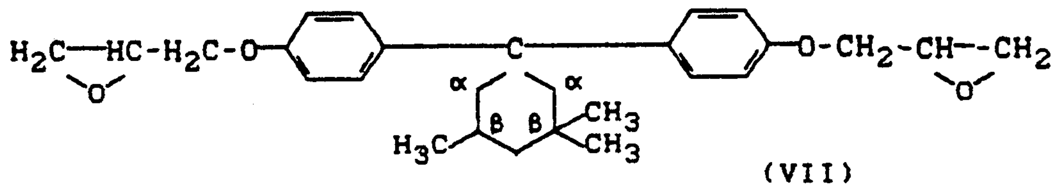

(VII)

Ansatz: 555 g (6 Mol) Epichlorhydrin
2 g Tetraethylammoniumchlorid
93 g (0,3 Mol) 1,1-Bis-(4-hydroxyphenyl)-3.3.5-trimethyl-cyclohexan (IV)
50,6 g (0,72 Mol) 45 %ige NaOH

In einem 2-Liter-3-Hals-Kolben, der mit Innenthermometer, Rührer und - über einen Wasserabscheider - einem Rückflußkühler versehen ist, wird das Epichlorhydrin, das Bisphenol der Formel (IV) und das Tetraethylammoniumchlorid 16 Stunden bei 60 °C unter $N_2$ zur Reaktion gebracht. Die anschließende Dehydrohalogenierung erfolgt innerhalb von 5 Stunden bei 130 mbar und 60 °C durch Zutropfen der Natronlauge unter gleichzeitigem Auskreisen des Wassers.

Nach der NaOH-Zugabe wird noch 2 Stunden bei den gleichen Bedingungen gerührt. Anschließend wird das Kochsalz abfiltriert, mit frischem Epichlorhydrin gewaschen und die vereinigten Filtrate bei 120 °C Badtemperatur und 13 mbar eingeengt. Das abdestillierte Epichlorhydrin wird für den nächsten Ansatz wiederverwendet. Der Rückstand ist ein bei Zimmertemperatur hochviskoses honigfarbenes Harz, das ohne weitere Reinigung verarbeitet wird.

Ausbeute: 122 g (96 % der Theorie)
% Epoxid MG 42: 19,75 gef.; 19,9 ber.; Restchlor: 0,7 %
$C_{27}H_{34}O_4$ (MG 422), IR, NMR und GC-MS Kopplung zeigen, daß die Verbindung in hoher Reinheit entstanden ist.

Beispiel 2

Hochmolekularer Diglycidylether aus Bisphenol der Formel IV und Diglycidylether der Formel VII (jeweils aus Bisphenol 1) im Molverhältnis 1:2
94 g (0,22 Mol) Isophorolidenbisphenoldiglycidether
31 g ( 0,1 Mol) Isophorolidenbisphenol

In einem Rundkolben wird der Diglycidylether der Formel VII vorgelegt und das Bisphenol der Formel III bei 150 °C unter Rühren zugegeben, exotherme Reaktion, Temperaturanstieg bis 185 °C, sowie Viskositätszunahme, deshalb bei 210 °C Innentemperatur unter Vakuum 1 Stunde nachgerührt.

Bei 210 °C auf Trockenblech gegeben, nach Erstarren bei Zimmertemperatur zerkleinert und abgefüllt.
Ausbeute: 125 g
ERW.-P.: 135 °C
% Epoxid: 7,3; 7,4 gef.; 7,3 ber.

Beispiel 3

59 Gew.-Teile des Diglycidylethers der Formel VII aus Beispiel 1 werden mit 42 Gew.-Teilen Methylhexahydrophthalsäureanhydrid und 0,5 Gew-Teilen Dimethylbenzylamin auf 80 °C erwärmt. Dabei entsteht ein flüssiges, homogenes Gemisch. Das Gemisch wird in eine Form gegossen (100 mm x 100 mm x 4 mm) 4 Stunden bei 80 °C, danach 16 Stunden bei 120 °C gehärtet. Aus dem erhaltenen Formkörper werden Prüfkörper geschnitten und deren mechanische Eigenschaften ermittelt.
Schlagzähigkeit (kJ/m$^2$) 3,8

Kugeldruckhärte (MPa) 177
Martensgrad (°C) 142

Beispiel 4

59 Gew.-Teile Diglycidylether der Formel VII aus Beispiel 1 werden mit 15,5 Gew.-Teilen Bis-(4-aminophenyl)sulfon bei 150°C gemischt, entgast und in eine Form gegossen (100 mm x 100 mm x 4 mm) und 5 Stunden bei bei 150°C, danach 20 Stunden bei 180°C gehärtet. Aus dem erhaltenen Formkörper werden Prüfkörper geschnitten und deren mechanische Eigenschaften ermittelt.
Schlagzähigkeit (kJ/$m^2$) 4,3
Kugeldruckhärte (MPa) 195
Martensgrad (°C) 200

Beispiel 5

100 Gew.-Teile des hochmolekularem Diglycidylesters aus Beispiel 2 werden mit 14,9 Gew.-Teilen Bis-(4-aminophenyl)-sulfon bei 180°C gemischt, entgast und in eine Form gegossen (100 mm x 100 mm x 4 mm) und 20 Stunden bei 200°C gehärtet. Aus dem erhaltenen Formkörper werden Prüfkörper geschnitten und deren mechanische Eigenschaften ermittelt.
Schlagzähigkeit (kJ/$m^2$) 13,7
Kugeldruckhärte (MPa) 175
Biegefestigkeit (MPa) 91
Randfaserdehnung (%) 3,7
Martensgrad (°C) 172

**Ansprüche**

1. Diglycidylverbindungen der Formel I

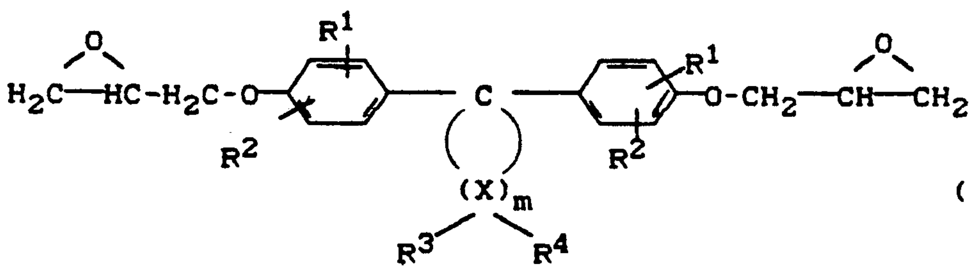

(I)

worin
$R^1$ und $R^2$ unabhängig voneinander Wasserstoff, Halogen, $C_1$-$C_8$-Alkyl, $C_5$-$C_6$-Cycloalkyl, $C_6$-$C_{10}$-Aryl und $C_7$-$C_{12}$-Aralkyl,
m eine ganze Zahl von 4 bis 7,
$R^3$ und $R^4$ für jedes X individuell wählbar, unabhängig voneinander Wasserstoff, $C_1$-$C_6$-Alkyl,
X Kohlenstoff bedeuten,
mit der Maßgabe, daß an mindestens einem Atom $R^3$ und $R^4$ gleichzeitig Alkyl ist.

2. Hochmolekulare Diglycidylverbindungen aus Diglycidylverbindungen der Formel I des Anspruchs 1 und Diphenolen.

3. 1,1-Bis-(4-epoxypropoxyphenyl)-3.3.5.-trimethylcyclohexan.

4. Verfahren zur Herstellung von Diglycidylverbindungen der Formel (I) des Anspruchs 1, dadurch gekennzeichnet, daß man gegebenenfalls ringsubstituierte Dihydroxydiphenylcycloalkane mit Epichlorhydrin in Gegenwart von Katalysatoren und Alkaliverbindungen in an sich bekannter Weise umsetzt.

5. Härtbare Mischungen, enthaltend Diglycidylverbindungen der Formel (I) des Anspruchs 1, gegebenenfalls andere Di-, Tri- und Tetraglycidylverbindungen und ein Härtungsmittel für Expoxidharze.

6. Härtbare Mischungen, enthaltend Diglycidylverbindungen der Formel (I) des Anspruchs 1, gegebenenfalls andere Di-, Tri- und Tetraglycidylverbindungen von Dihydroxydiphenyl-cycloalkanen sowie gegebenenfalls bekannte Härter.

Europäisches Patentamt

# EUROPÄISCHER RECHERCHENBERICHT

Nummer der Anmeldung

EP 89124179.6

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int Cl 5) |
|---|---|---|---|
| A | EP - A1 - 0 224 391 (MITSUI SEKIYU KAGAKU KOGYO KABUSHIKI KAISHA) * Zusammenfassung * -- | 1,4-6 | C 07 D 303/30<br>C 08 G 59/04 |
| A | CHEMICAL ABSTRACTS, Band 104, Nr. 4, 27. Jänner 1986, Columbus, Ohio, USA DAIKIN KOGYO CO. "Carriers for electrostatographic developers" Seite 472, Spalte 1, Zusammenfassung-Nr. 26 758u & Jpn. Kokai Tokkyo Koho JP 60 60 657 (85 60 6573) -- | 1,4-6 | |
| A | CHEMICAL ABSTRACTS, Band 103, Nr. 18, 4. November 1985, Columbus, Ohio, USA HITACHI CHEMICAL CO., LTD. "Epoxy resin compositions" Seite 36, Spalte 1, Zusammenfassung-Nr. 142 894p & Jpn. Kokai Tokkyo Koho JP 60 69 125 (85 69 125) -- | 1,4-6 | **RECHERCHIERTE SACHGEBIETE (Int. Cl 5)**<br>C 07 D 303/00<br>C 08 G 59/00 |
| A | CHEMICAL ABSTRACTS, Band 103, Nr. 24, 16. Dezember 1985, Columbus, Ohio, USA KONISHIROKU PHOTO INDUSTRY CO. LTD. "Carriers for electro-static image developers" Seite 521, Spalte 1, Zusammenfassung-Nr. 203 705b & Jpn. Kokai Tokkyo Koho JP 60 60 660 (85 60 660) -- | 1,4-6 | |
| A | CHEMICAL ABSTRACTS, Band 103, Nr. 24, 16. Dezember 1985, | 1,4-6 | |

Der vorliegende Recherchenbericht wurde für alle Patentansprüche erstellt.

| Recherchenort | Abschlußdatum der Recherche | Prüfer |
|---|---|---|
| WIEN | 13-03-1990 | BRUS |

KATEGORIE DER GENANNTEN DOKUMENTEN
X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur
T : der Erfindung zugrunde liegende Theorien oder Grundsätze

E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument

EPA Form 1503 03 82

Europäisches Patentamt

# EUROPÄISCHER RECHERCHENBERICHT

Nummer der Anmeldung

-2-
EP 89124179.6

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int Cl 5) |
|---|---|---|---|
| | Columbus, Ohio, USA KONISHIROKU PHOTO INDUSTRY CO. LTD. "Carriers for electrostatographic developers" Seite 521, Spalte 1, Zusammenfassung-Nr. 203 706c & Jpn. Kokai Tokkyo Koho JP 60 60 659 (85 60 659) -- | | |
| A | CHEMICAL ABSTRACTS, Band 103, Nr. 24, 16. Dezember 1985, Columbus, Ohio, USA KONISHIROKU PHOTO INDUSTRY CO. LTD. "Carriers for electrographic developers" Seite 521, Spalte 1, Zusammenfassung-Nr. 203 707d & Jpn. Kokai Tokkyo Koho JP 60 60 656 (85 60 656) ---- | 1,4-6 | |
| | | | RECHERCHIERTE SACHGEBIETE (Int. Cl 5) |

Der vorliegende Recherchenbericht wurde für alle Patentansprüche erstellt.

| Recherchenort | Abschlußdatum der Recherche | Prüfer |
|---|---|---|
| WIEN | 13-03-1990 | BRUS |

KATEGORIE DER GENANNTEN DOKUMENTEN
X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur
T : der Erfindung zugrunde liegende Theorien oder Grundsätze

E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument

EPA Form 1503 03 82